# EUROPEAN PATENT APPLICATION

(11) **EP 2 053 529 A2**
(43) Date of publication of application: **29.04.2009**
(21) Application number: 08162205.2
(22) Date of filing: 12.08.2008
(51) Int. Cl.: G06F 19/00

(54) **Health information collecting apparatus, management apparatus, health information collecting system, and method for collecting health information**

(30) Priority: 19.10.2007 JP 2007272116
(71) Applicant: Panasonic Corporation, Kadoma-shi Osaka 571-8501 (JP)
(72) Inventor: Tajika, Yosuke, Shiromi, Chuo-ku Osaka, Osaka-shi 540-6207 (JP); Aizu, Kazuhiro, Shiromi, Chuo-ku Osaka, Osaka-shi 540-6207 (JP); Nishiyama, Hiromichi, Shiromi, Chuo-ku Osaka, Osaka-shi 540-6207 (JP); Kobayashi, Daisuke, Shiromi, Chuo-ku Osaka, Osaka-shi 540-6207 (JP)
(74) Representative: Pautex Schneider, Nicole Véronique

(57) **Abstract**

The health information collecting apparatus according to the present invention includes: a clock unit configured to oscillate a clock; a counting unit configured to count the clock oscillated by the clock unit; an obtainment unit configured to obtain the health information from the measuring device; a holding unit configured to hold the health information and a first clock count associated with the health information, the first clock count being counted by the counting unit when the health information is received; and a transmission unit configured to transmit, to the management apparatus, the health information and the first clock count, wherein the transmission unit transmits, to the management apparatus, a second clock count with the health information and the first clock count, the second clock count being counted by the counting unit when the health information and the first clock count are transmitted to the management apparatus.

## Description

### BACKGROUND OF THE INVENTION

### (1) Field of the Invention

The present invention relates to health information collecting apparatuses that collect, as health information, biological data obtained through measurement using a sensor, management apparatuses, health information collecting systems, and methods for collecting health information. In particular, the present invention relates to a health information collecting apparatus that collects health information using a battery-driven wearable terminal having low operation specifications and no real time clock, a management apparatus, a health information collecting system, and a method for collecting health information.

### (2) Description of the Related Art

In recent years, people have been increasingly concerned about health. In response, commercial services have been emerging that collect, as health information, biological data obtained using a sensor and provide health advice on exercise, diet, and so on using the collected health information.

There has been proposed a health information collecting system in which a portable memory device collects and accumulates health information wirelessly transmitted from a sensor, and further transmits, with an external wake-up call, the health information to a wireless base station (for example, see FIG. 3 in Patent Reference 1: Japanese Unexamined Patent Application Laid-Open Publication No. 2006-262993).

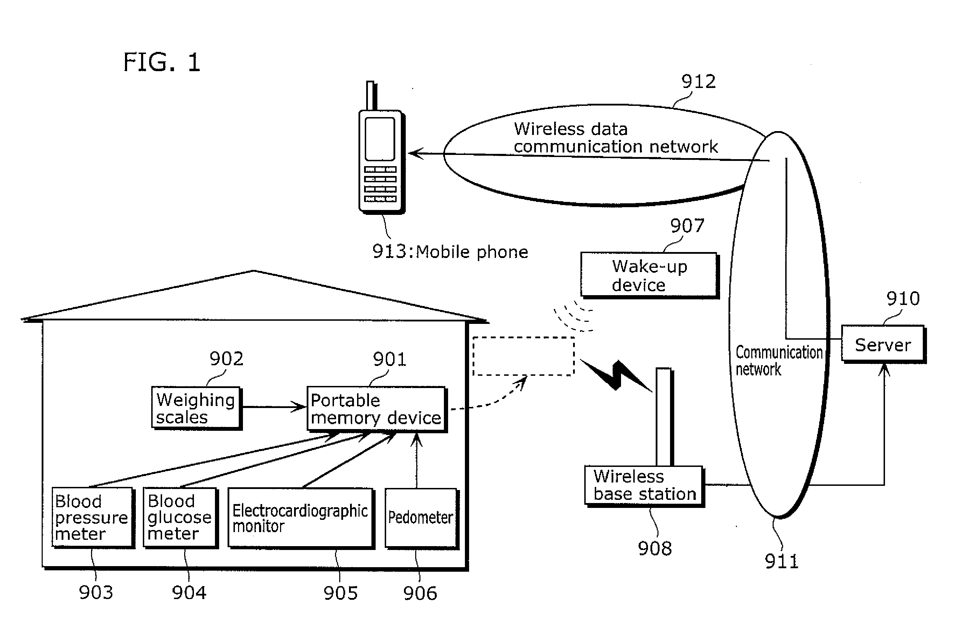

FIG. 1 illustrates an overview of a conventional health information collecting system.

In FIG. 1, a portable memory device 901 obtains, through wire or wirelessly, health information from respective sensors such as a weighing scales 902, a blood pressure meter 903, a blood glucose meter 904, an electrocardiographic monitor 905, and a pedometer 906, and stores the obtained health information therein.

Here, a wake-up device 907 transmits a wake-up signal to the portable memory device 901. Upon receiving the wake-up signal, the portable memory device 901 transmits the stored health information to a wireless base station 908. The health information transmitted to the wireless base station 908 is transmitted to a server 910 via a communication network 911.

It is to be noted that the health information transmitted to the server 910 can be checked with a mobile phone 913 via a wireless data communication network 912.

Furthermore, there has been proposed a conventional health information collecting system in which health information obtained using a biological information measuring device worn by a user is wirelessly transmitted to and collected in a server (for example, see Patent Reference 2: Japanese Unexamined Patent Application Laid-Open Publication No. 2005-329111).

### SUMMARY OF THE INVENTION

The following problem, however, arises with the configurations of the above-mentioned health information collecting systems. That is to say, for example, in the case where there is a time from when a portable memory device such as a battery-driven wearable terminal having low operation specifications and no real time clock has received health information from a sensor to when the portable memory device is to transmit the health information to a wireless base station, it is impossible to know at what point of time the transmitted health information was obtained by the sensor.

The present invention has been devised to solve the above-mentioned problem.

In order to achieve the above-mentioned object, the health information collecting apparatus according to one aspect of the present invention which obtains health information from a measuring device and transmits the obtained health information to a management apparatus, the measuring device measuring the health information of a user of the measuring device, the management apparatus managing the transmitted health information, the health information collecting apparatus including: a clock unit configured to oscillate a clock; a counting unit configured to count the clock oscillated by the clock unit; an obtainment unit configured to receive the health information from the measuring device; a holding unit configured to hold the health information and a first clock count associated with the health information, the first clock count being counted by the counting unit when the health information is received; and a transmission unit configured to transmit, to the management apparatus, the health information and the first clock count associated with the heal information, wherein the transmission unit is configured to transmit, to the management apparatus, a second clock count with the health information and the first clock count, the second clock count being counted by the counting unit when the health information and the first clock count are transmitted to the management apparatus.

According to the present invention, it is possible to determine the obtainment time of the health information collected using the battery-driven wearable terminal having low operation specifications and no real time clock. In particular, according to the health information collecting apparatus, the management apparatus, and the health information collecting system, and the method for collecting health information of the present invention, it is possible to determine the obtainment time of the health information collected using the wearable terminal having no capability of time management and low operations specifications.

### FURTHER INFORMATION ABOUT TECHNICAL BACKGROUND TO THIS APPLICATION

The disclosure of Japanese Patent Application No. 2007-272116 filed on October 19, 2007 including specification, drawings and claims is incorporated herein by reference in its entirety.

### BRIEF DESCRIPTION OF THE DRAWINGS

These and other objects, advantages and features of the invention will become apparent from the following description thereof taken in conjunction with the accompanying drawings that illustrate a specific embodiment of the invention. In the drawings:
FIG. 1 illustrates an overview of a conventional health information collecting system;
FIG. 2 illustrates an overview of a health information collecting system according to the present invention;
FIG. 3 is a block diagram illustrating a hardware configuration example of a wearable health-care recorder (WHR) in the health information collecting system according to the present invention;
FIG. 4 is a block diagram illustrating a hardware configuration example of a management apparatus in the health information collecting system according to the present invention;
FIG. 5 illustrates WHR-management apparatus communication procedures at body temperature measurement according to a first embodiment of the present invention;
FIG. 6 is a flow chart describing operations of the WHR according to the first embodiment of the present invention;
FIG. 7 illustrates a status of data stored in a memory of the WHR according to the first embodiment of the present invention;
FIG. 8 illustrates a data frame at a time when the WHR transmits health information to the management apparatus according to the first embodiment of the present invention;
FIG. 9 is a flow chart describing operations of the management apparatus according to the first embodiment of the present invention;
FIG. 10 illustrates conversion of a clock count at body temperature measurement to time according to the first embodiment of the present invention;
FIG. 11 illustrates setting of a terminal ID to a weighing scales according to a second embodiment of the present invention;
FIG. 12 is a block diagram illustrating a configuration in the case where a SD card is inserted and attached to the weighing scales according to the second embodiment of the present invention;
FIG. 13 illustrates WHR-weighing scales communication procedures at weight measurement according to the second embodiment of the present invention; and
FIG. 14 illustrates one example of information compression at pedometer measurement according to the present invention.

### DECRIPTION OF THE PREFERRED EMBODIMENT(S)

The health information collecting apparatus according to a first aspect of the present invention is a health information collecting apparatus which obtains health information from a measuring device and transmits the obtained health information to a management apparatus, the measuring device measuring the health information of a user of the measuring device, the management apparatus managing the transmitted health information, the health information collecting apparatus including: a clock unit configured to oscillate a clock; a counting unit configured to count the clock oscillated by the clock unit; an obtainment unit configured to receive the health information from the measuring device; a holding unit configured to hold the health information and a first clock count with the health information, the first clock count being counted by the counting unit when the health information is received; and a transmission unit configured to transmit, to the management apparatus, the health information and the first clock count associated with the health information, wherein the transmission unit is configured to transmit, to the management apparatus, a second clock count together with the health information and the first clock count, the second clock count being counted by the counting unit when the health information and the first clock count are transmitted to the management apparatus.

According to the first aspect of the present invention, even in the case where the health information collecting apparatus does not include a real time clock unit, the health information collecting apparatus transmits, to the management apparatus, the second clock count at a time when the health information collecting apparatus transmits the health information and the first clock count, with the health information that is associated with the first clock count and the first clock count at a time when the health information collecting apparatus receives the health information. The management apparatus determines a measurement time of measuring the health information by determining real time corresponding to the second clock count based on real time counted by the real time clock unit at a time when the management apparatus receives the health information from the health information collecting apparatus, and by determining real time associated with the first clock count based on the second clock count whose real time is determined. Accordingly, the structure of the health information collecting apparatus can be simplified, and a physical and mental strain on the human body when the health information collecting apparatus is carried can be reduced.

In addition, in the case where the measuring device measures the health information several times, the health information collecting apparatus receives the health information from the measuring device every time the measuring device measures the health information. Although, every time the health information collecting apparatus receives the health information from the measuring device, the health information collecting apparatus may transmit, to the management apparatus, the health information and the first clock count at a time when the health information is received from the measuring device, the health information collecting apparatus may also transmit plurality of sets of the health information and the first clock count all together to the management apparatus, the first clock count being associated with the health information in each of the sets. Transmitting the plurality of sets of the health information and the first clock count all together to the management apparatus simplifies processing as a whole, and an accurate measurement time of each of plural health information measured can be calculated even with the simplified processing. For instance, even in the case where a body temperature is measured every one hour and measured body temperature data is transmitted to the management apparatus after every measurement and even in the case where plurality of sets of body temperature data measured in a day are transmitted all together to the management apparatus, an accurate measurement time of each body temperature data can be determined.

Moreover, the health information collecting apparatus according to the first aspect of the present invention may include the transmission unit configured to transmit, to the management device, the health information and the first clock count, with clock cycle information indicating a time interval of a clock cycle at which the clock unit oscillates the clock.

According to the first aspect of the present invention, even when the management apparatus knows beforehand the time interval of the clock cycle of the health information collecting apparatus, it is possible to know the time interval by obtaining the health information. Since this allows the management apparatus to determine the real time corresponding to the first clock count based on the second clock count whose real time is determined, it is possible to determine the measurement time of the health information.

Furthermore, the health information collecting apparatus according to the first aspect of the present invention may include a carrier sense unit configured to detect a radio wave, wherein the transmission unit is configured to transmit the health information and the first clock count with the second clock count, when the management apparatus requests the health information collecting apparatus for health information and when the carrier sense unit detects a radio wave from the management apparatus.

According to the first aspect of the present invention, when the health information collecting apparatus does not establish communication with the management apparatus, it is possible to reduce power consumption by performing an intermittent operation. This allows extension of a power source life in which the health information collecting apparatus can operate.

Moreover, the health information collecting apparatus according to the first aspect of the present invention may include the obtainment unit configured to request the measuring device to transmit, to the health information collecting apparatus, the health information measured by the measuring device, and to receive, from the measuring device, the health information.

According to the first aspect of the present invention, since it is possible to reduce a time lag between timing at which the measuring devices measures the health information and timing at which the health information collecting apparatus obtains the health information from the measuring device, it becomes possible to accurately calculate time when the measuring device measured the health information.

Furthermore, the health information collecting apparatus according to the first aspect of the present invention may include the obtainment unit configured to request the measuring device for the health information, when the measuring device detects the health information collecting apparatus and when the carrier sense unit detects the radio wave from the measuring device.

According to the first aspect of the present invention, when the health information collecting apparatus does not establish the communication with the management apparatus, it is possible to reduce the power consumption by performing the intermittent operation. This allows extension of a power source life in which the health information collecting apparatus can operate.

Moreover, the health information collecting apparatus according to the first aspect of the present invention may include: the holding unit configured to hold a plurality of sets of the health information and the first clock count, and to hold an interspace of time as the first clock count associated with health information, the interspace of time being a difference between a previous clock count by the counting unit at a time when previous health information is received by the health information collecting apparatus and a current clock count counted by the counting unit at a time when current health information is received by the health information collecting apparatus; and the transmission unit configured to transmit, to the management apparatus, with the plurality of sets of the health information and the interspace of time as the first clock count, a clock count as the second clock count, the clock count being counted by the counting unit at a time when the plurality of sets of the health information and the first clock count are transmitted to the management apparatus.

According to the first aspect of the present invention, the health information collecting apparatus transmits, to the management apparatus, the health information, the second clock at a time when the health information collecting apparatus transmits the health information with the first clock count to the management apparatus, and, instead of the first clock at a time when the health information collecting apparatus obtains the health information from the measuring device, information indicating a difference between the first clock count and the second clock count. Based on the second clock count, the management apparatus can calculate the measurement time of the health information from the information indicating the difference. As a result, since an amount of data which the health information collecting apparatus transmits to the management apparatus can be reduced, a processing load in the health information collecting apparatus and the management apparatus can be reduced. Accordingly, the structure of the health information collecting apparatus can be simplified, and a physical and mental strain on the human body when the health information collecting apparatus is carried can be reduced.

Furthermore, the health information collecting apparatus according to the first aspect of the present invention may include: a storage capacity detection unit configured to detect a remaining storage capacity of the holding unit; and an information compression unit configured to compress specific sets of the health information and the first clock count among the sets of the health information and the first clock count, wherein the information compression unit is configured to compress the specific sets of the health information and the first clock count, when the storage capacity detection unit detects that the remaining storage capacity is equal to or less than a predetermined value.

According to the first aspect of the present invention, since it is possible to compress plural health information in the health information collecting apparatus, it becomes possible to use a limited storage capacity effectively.

Moreover, the health information collecting apparatus according to the first aspect of the present invention may include the transmission unit configured to transmit, to the management apparatus, the second clock count with the health information and a third clock count as the first clock count, the third clock count being a difference between the first clock count, the second clock count and the second clock count being the basis for calculating the difference.

According to the first aspect of the present invention, even in the case where the health information collecting apparatus does not include the real time clock unit, the health information collecting apparatus transmits, to the management apparatus, the health information, the second clock at a time when the health information collecting apparatus transmits the health information with the first clock count to the management apparatus, and, instead of the first clock at a time when the health information collecting apparatus obtains the health information from the measuring device, information indicating a difference between the first clock count and the second clock count. Based on the second clock count, the management apparatus can calculate the measurement time of the health information from the information indicating the difference. Since it is possible to reduce the amount of data which the health information collecting apparatus transmits to the management apparatus by transmitting the information indicating the difference instead of the first clock count to the management apparatus, the processing load in the management apparatus can be reduced. Accordingly, the structure of the health information collecting apparatus can be simplified, and a physical and mental strain on the human body when the health information collecting apparatus is carried can be reduced.

Furthermore, the management apparatus according to a second aspect of the present invention includes: a communication unit configured to receive the health information, a first clock count, and second clock count from the health information collecting apparatus, the first clock count being a clock count value at a time when the health information collecting apparatus collects the health information from the measuring device and the second clock count being another clock count value at a time when the health information collecting apparatus transmits, to the management apparatus, the health information and the first clock count associated with the health information; a holding unit configured to hold the health information, the first clock count associated with the health information, and the second clock count; a time determination unit configured to determine a reception time of receiving the second clock count; and a conversion unit configured to convert the first clock count to actual time using a difference between the first clock count and the second clock count, based on the reception time determined by the time determination unit, and to hold the actual time with the health information.

According to the second aspect of the present invention, even in the case where the health information collecting apparatus does not include the real time clock unit, the management apparatus receives, from the health information collecting apparatus, the first clock count at a time when the health information collecting apparatus obtains the health information, the first clock count associated with the health information, the second clock count at a time when the information collecting apparatus transmits the health information, thereby calculating the measurement time of the health information based on the first clock count, the second clock count, and the real time clock unit included in the management apparatus. Accordingly, the structure of the health information collecting apparatus can be simplified, and a physical and mental strain on the human body when the health information collecting apparatus is carried can be reduced.

Moreover, the management apparatus according to a second aspect of the present invention includes: the communication unit configured to receive, from the health information collecting apparatus, the health information, the second clock count, and a third clock count as the first clock count, the third clock count being a difference between the first clock count and the second clock count, the second clock count being the basis for calculating the difference; and the conversion unit configured to convert the third clock count to actual time using the difference, based on the reception time of receiving the second clock count determined by the time determination unit, and to hold the actual time with the associated health information.

According to the second aspect of the present invention, even in the case where the health information collecting apparatus does not include the real time clock unit, the management apparatus receives, from the health collecting apparatus, the health information, the second clock at a time when the health information collecting apparatus transmits the health information with the first clock count to the management apparatus, and, instead of the first clock at a time when the health information collecting apparatus obtains the health information from the measuring device, information indicating a difference between the first clock count and the second clock count. Based on the second clock count, the management apparatus can calculate the measurement time of the health information from the information indicating the difference. Accordingly, the structure of the health information collecting apparatus can be simplified, and a physical and mental strain on the human body when the health information collecting apparatus is carried can be reduced.

In addition, since it is possible to reduce the amount of data which the management apparatus receives by receiving the information indicating the difference, the processing load in the management apparatus can be reduced.

It is to be noted that the above-mentioned aspects of the present invention can be, for example, realized as a device, as an integrated circuit including processing measures included in such a device, as a method having the processing measures included in the device as steps, and as a program causing a computer to execute the steps. The program may be distributed via recording media such as CD-ROM and via communication media such as the Internet.

The following describes embodiments of the present invention with reference to the drawings.

### (First Embodiment)

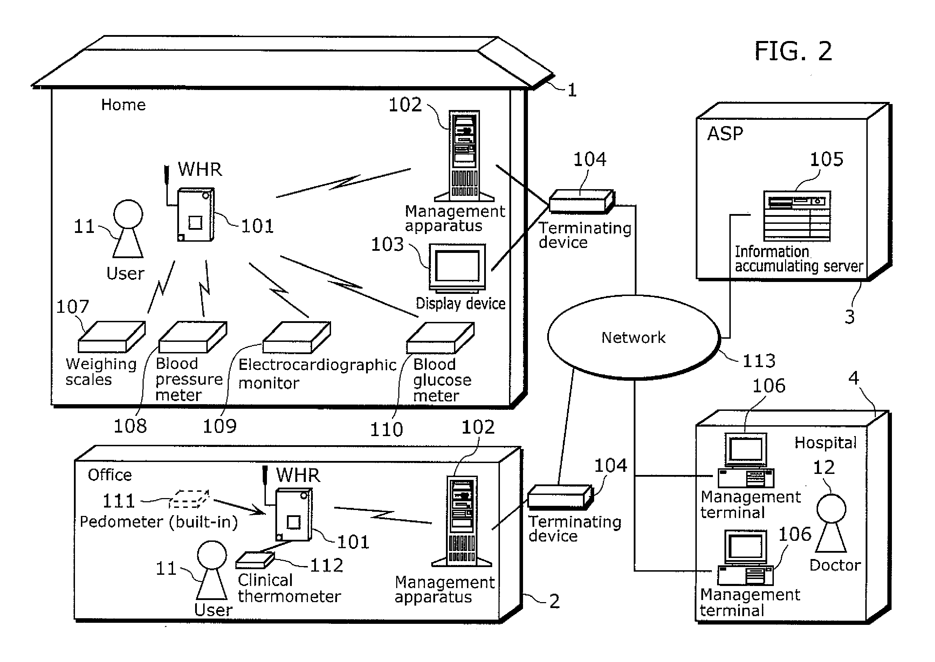

FIG. 2 illustrates an overview of a health information collecting system according to the present invention.

A wearable health-care recorder (WHR) 101 is a wearable terminal carried or worn by a user 11.

The WHR 101 is a health information collecting apparatus that collects, as health information, data on a body of the user 11 obtained through measurement with a variety of sensors such as a weighing scales 107, a blood pressure meter 108, an electrocardiographic monitor 109, a blood glucose meter 110, a pedometer 111, and a clinical thermometer 112.

Moreover, the WHR 101 having a wireless communication function transmits and receives health information and control information through wireless communication with a management apparatus 102.

Furthermore, since the WHR 101 is battery-driven and has low operation specifications and no real time clock, the WHR 101 contains no real time information. However, the WHR 101 can record, instead of the real time information, a unique clock counted by an internal timer.

It is to be noted that the communication between the WHR 101 and the variety of sensors may take diverse forms. For example, the WHR 101 may wirelessly communicate with a sensor such as the weighing scales 107, the blood pressure meter 108, the electrocardiographic monitor 109 or the blood glucose meter 110, communicate with the clinical thermometer 112 through wire, and internally communicate with the pedometer 111 in the case where the WHR 101 has the built-in pedometer 111.

The WHR 101 performs an intermittent operation consisting of a carrier sense operation for detecting an activation command from the variety of sensors or from the management apparatus 102 and a suspended state for reducing power consumption. Here, the carrier sense operation is a mechanism for checking a radio wave status and checking again a radio wave status after a certain period of time when the management apparatus 102 has not transmitted an activation command such as a search request. Furthermore, the activation command is an electric text for requesting the WHR 101 to activate. The command may be also referred to as a message.

In addition, the WHR 101 obtains the health information from the variety of sensors according to a predetermined cycle or an event operated by the user 11.

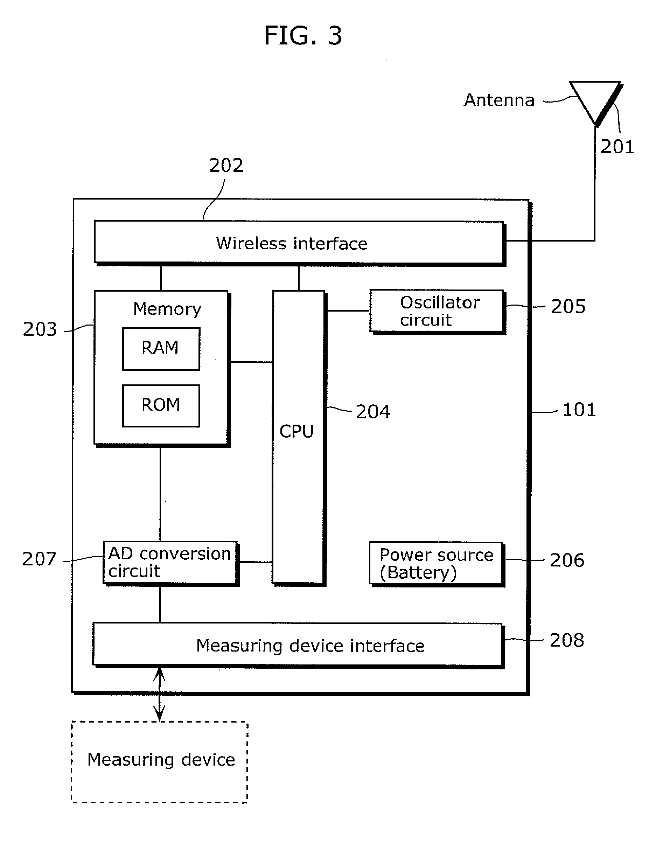

FIG. 3 is a block diagram illustrating a hardware configuration example of the WHR 101 in the health information collecting system according to the present invention.

As shown in FIG. 3, the WHR 101 includes an antenna 201, a wireless interface 202, a memory 203, a CPU 204, an oscillator circuit 205, a power source 206, an AD conversion circuit 207, and a measuring device interface 208.

The wireless interface 202 communicates with the management apparatus 102 via the antenna 201. It is to be noted that the wireless interface 202 may obtain, via the antenna 201, the health information from the variety of sensors, that is, measuring devices such as the weighing scales 107, the blood pressure meter 108, the electrocardiographic monitor 109, the blood glucose meter 110, the pedometer 111, and the clinical thermometer 112.

The memory 203 includes, for example, a random access memory (RAM) and read only memory (ROM), and the health information obtained from the variety of sensors such as the weighing scales 107, the blood pressure meter 108, the electrocardiographic monitor 109, the blood glucose meter 110, the pedometer 111, and the clinical thermometer 112 is stored in the memory 203. Here, together with a clock count at a time when the health information is obtained (hereinafter, described as a clock count at measurement), the health information is stored in the memory 203.

The CPU 204 controls the wireless interface 202, the memory 203, the oscillator circuit 205, and the AD conversion circuit 207.

The oscillator circuit 205, equivalent to an internal timer, generates a clock.

The power source 206 includes, for example, a battery such as a battery cell.

The AD conversion circuit 207 performs A/D conversion on the health information obtained from the variety of sensors such as the weighing scales 107, the blood pressure meter 108, the electrocardiographic monitor 109, the blood glucose meter 110, the pedometer 111, and the clinical thermometer 112.

The measuring device interface 208 is an interface used for obtaining the health information from the variety of sensors such as the weighing scales 107, the blood pressure meter 108, the electrocardiographic monitor 109, the blood glucose meter 110, the pedometer 111, and the clinical thermometer 112.

As shown in FIG. 2, the management apparatus 102 obtains the health information from the WHR 101 and controls operations of the WHR 101. The management apparatus 102 is connected to a network 113 via a terminating device 104.

Furthermore, the management apparatus 102 has resources equal to a personal computer (PC), a set-top box (STB) and the like, and holds real time information.

The management apparatus 102 wirelessly transmits an activation command to the WHR 101 periodically or when operated by the user 11. In the case where the WHR 101 responses to the activation command, the management apparatus 102 obtains the health information from the WHR 101 and controls the operations of the WHR 101. When the management apparatus 102 obtains the health information from the WHR 101, the management apparatus 102 calculates obtainment time of obtaining the health information based on the clock count at measurement contained in a wireless message of the WHR 101. Then, the management apparatus 102 transmits the obtained health information to an information accumulating server 105 via the terminating device 104 and the network 113.

It is to be noted that when the management apparatus 102 is not connected to the terminating device 104 and the network 113, the management apparatus 102 may process the health information locally and, in such a case, hold the health information therein.

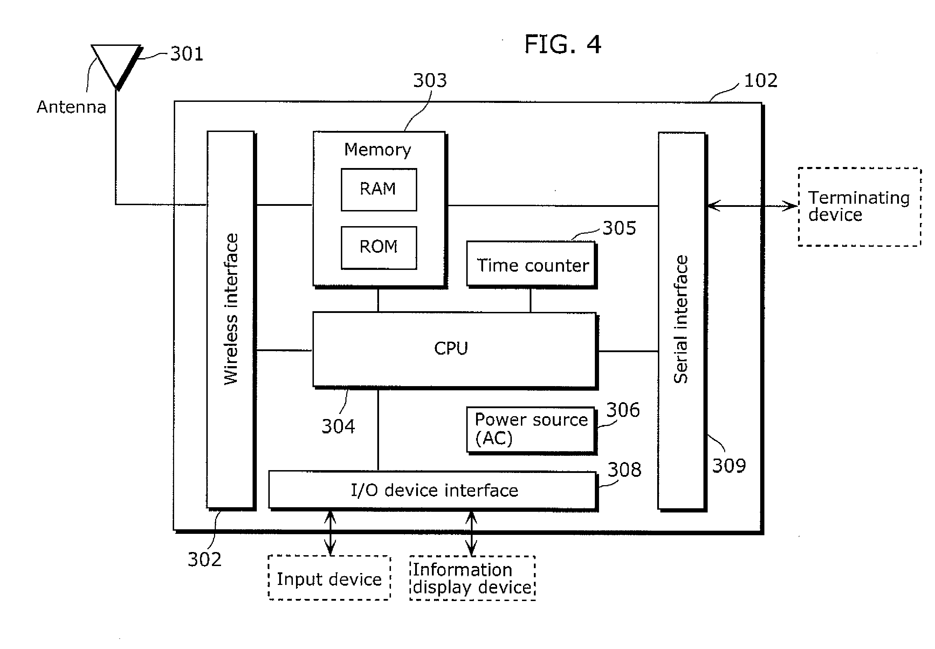

FIG. 4 is a block diagram illustrating a hardware configuration example of the management apparatus 102 in the health information collecting system according to the present invention.

As shown in FIG. 4, the management apparatus 102 includes an antenna 301, a wireless interface 302, a memory 303, a CPU 304, a time counter 305, a power source 306, an I/O device interface 308, and a serial interface 309.

The wireless interface 302 communicates with the WHR 101 via the antenna 301.

The memory 303 includes, for example, a RAM and ROM, and stores the health information obtained from the WHR 101. Here, the health information obtained from the WHR 101 contains a clock count at a time when the WHR 101 transmits the health information to the management apparatus 102 (hereinafter, referred to as a clock count at transmission) in addition to the clock count at measurement. Moreover, together with the health information containing the clock count at transmission, time when the management apparatus 102 obtains the health information from the WHR 101 (hereinafter, described as current time) is stored in the memory 303.

The CPU 304 controls the wireless interface 302, the memory 303, the time counter 305, the I/O device interface 308, and the serial interface 309.

The time counter 305 has an oscillator circuit, causes the oscillator circuit to generate a clock, and counts time using the clock.

The power source 306 includes, for example, a domestic alternator such as an alternating-current (AC) power.

The I/O device interface 308 is, for example, an interface to which an operation of a user is inputted via an input device such as a keyboard. In addition, the I/O device interface 308 is, for example, an interface which outputs contents to be displayed on an information display device such as a display device 103.

The serial interface 309 is an interface connecting to the terminating device 104. The serial interface 309 is used for transmitting the health information stored in the memory 303 to the information accumulating server 105 via the terminating device 104 and the network 113.

The terminating device 104 is a device functioning as a gateway connecting a local network including the management apparatus 102 and the display device 103 to the external network 113. It is to be noted that the terminating device 104 may be realized as one function of the management apparatus 102.

The network 113 is a commonly used shared network. For the connection configuration of the network, for example, in addition to a network configuration through wire such as a telephone network and optical cable network, a wireless network configuration such as a mobile phone network and the Worldwide Interoperability for Microwave Access (WiMAX) may be used.

The information accumulating server 105 is set up in an application service provider (ASP) 3, and accumulates health information transmitted from management apparatuses 102 of each household and office. Furthermore, the information accumulating server 105 transmits necessary health information according to access contents from the management apparatus 102 and a management terminal 106.

For instance, sensors such as the weighing scales 107, the blood pressure meter 108, the electrocardiographic monitor 109, and the blood glucose meter 110 are in a household 1 shown in FIG. 2. Measurement using these sensors enables biological data of the user 11 to be obtained as health information. Here, the variety of sensors such as the weighing scales 107, the blood pressure meter 108, the electrocardiographic monitor 109, and the blood glucose meter 110 have, for example, a wireless communication function in addition to a measurement function such as weight measurement, and can wirelessly communicate with the WHR 101 using the wireless communication function.

First, the user carrying the WHR 101 performs measurement with one of the sensors. The sensor transmits health information obtained by measuring the user 11 to the WHR 101. The WHR 101 holds the health information received from the sensor together with a clock count at measurement.

Next, when, with the WHR 101 being carried, the user 11 nears the management apparatus 102, the WHR 101 receives an activation command transmitted by the management apparatus 102 periodically or when operated by the user 11. When, after receiving the activation command, the WHR 101 further receives a transmission command from the management apparatus 102, the WHR 101 transmits the held health information together with a clock count at transmission to the management apparatus 102.

Besides converting the health information containing the clock count at measurement and the clock count at transmission received from the WHR 101 into health information containing real time information, the management apparatus 102 transmits the health information containing the real time information to the information accumulating server 105 via the terminating device 104 and the network 113. Subsequently, the information accumulating server 105 accumulates the transmitted health information containing the real time information.

Moreover, an operation of the management apparatus 102 by the user 11 causes the management apparatus 102 to access the information accumulating server 105 and to obtain contents according to health service provided by the ASP 3, and the obtained contents can be displayed on the display device 103.

Furthermore, for example, in an office 2 shown in FIG. 2, the user 11 carries the WHR 101 having the built-in pedometer 111. As shown in FIG. 2, the user 11 also uses the clinical thermometer 112. Here, the clinical thermometer 112 is connected to the WHR 101 through wire.

The WHR 101 holds pedometer data (the number of steps taken) obtained from the pedometer 111 included in the WHR 101 as health information. In addition, the WHR 101 obtains body temperature data of the user 11 measured by the clinical thermometer 112 through wire communication, and holds the obtained body temperature data as health information.

Similar to the household 1, the management apparatus 102 is in the office 2, and collects health information through wireless communication with the WHR 101.

Furthermore, for example, in a hospital 4 shown in FIG. 2, a doctor 12 accesses the information accumulating server 105 using the management terminal 106, and obtains health information of the user 11 during an examination of the user 11. Here, the doctor 12 obtains the health information of the user 11 by accessing the information accumulating server 105 using a terminal ID of the WHR 101 carried by the user 11 or personal information of the user 11. Due to the obtained health information, the doctor 12 can know a health condition of the user 11 prior to visiting the hospital 4.

It is to be noted that a communication function with the WHR 101 may be added to the management terminal 106, and the health information held by the WHR 101 may be directly displayed by the management terminal 106.

### <Communication Procedures>

The following describes an example of collecting, as health information, body temperature data measured using the clinical thermometer 112 as one of the sensors. Here, the clinical thermometer 112 is connected to the WHR 101 through wire or wirelessly.

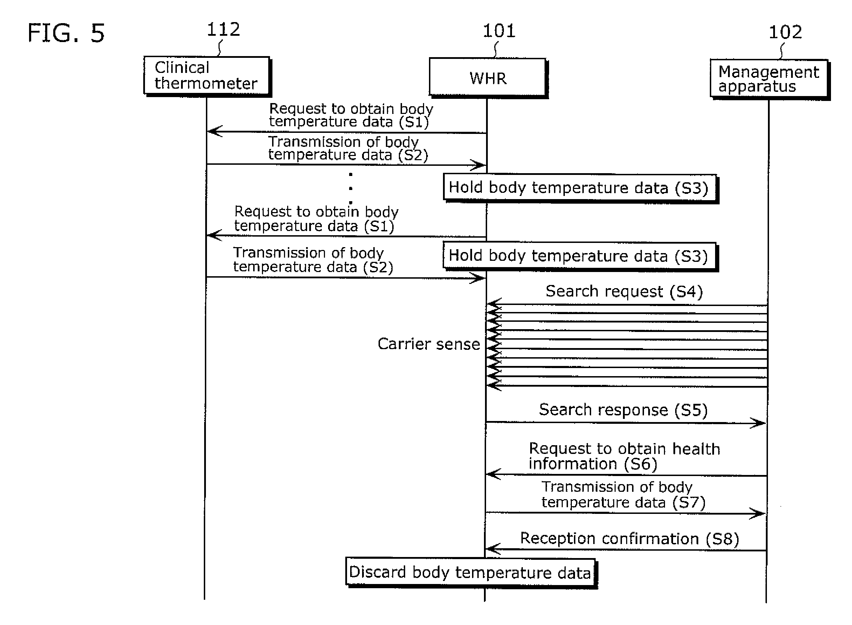

FIG. 5 illustrates WHR 101-management apparatus 102 communication procedures at body temperature measurement according to the first embodiment of the present invention. In FIG. 5, the same elements as in FIG. 2 are indicated by the same reference numerals, and their description is omitted.

When measuring body temperature data, the WHR 101 first transmits, to the clinical thermometer 112, a command for request to obtain body temperature data (S1). The clinical thermometer 112 receives the command for request to obtain body temperature data and measures a body temperature of the user 11.

Next, the clinical thermometer 112 transmits, to the WHR 101, the body temperature data obtained through measurement of the body temperature of the user 11 (S2).

It is to be noted that in the case where the WHR 101 has the built-in clinical thermometer 112, the body temperature data may be obtained without communication procedures of Steps S1 and S2.

In addition, even when the WHR 101 does not transmit, to the clinical thermometer 112, the command for request to obtain body temperature data, the clinical thermometer 112 may transmit the body temperature data to the WHR 101.

Next, the WHR 101 causes a storage memory 203 to store the body temperature data received from the clinical thermometer 112. At this time, the WHR 101 holds the body temperature data by storing, in the storage memory 203, a clock counted by the internal timer (a clock count at measurement), in association with the body temperature data (S3). These operations described in Steps S1 to S3 are performed every time the body temperature is measured.

Except when measuring the body temperature data, the WHR 101 performs the intermittent operation in which a radio wave carrier sense operation and a suspended state in turn are periodically repeated. The WHR 101 avoids wireless transmission for power saving as long as the WHR 101 does not receive any wireless messages, and can operate for many hours even with a battery.

In addition, the management apparatus 102 transmits a command for search request as an activation command periodically or when operated by the user.

Next, the WHR 101 searches whether a search request from the management apparatus 102 is present by performing the carrier sense operation (S4). When the search request is absent, the intermittent operation is performed. That is to say, the carrier sense operation is performed again after waiting ready for a certain period of time.

Here, the search request from the management apparatus 102 is continuously transmitted for a period of time longer than a period of time when the WHR 101 waits ready. This allows the search request to be detected surely by the carrier sense operation in the case where the WHR 101 is located within a wirelessly reachable range of the management apparatus 102.

When the WHR 101 receives the search request from the management apparatus 102 and judges that it is a relevant command, the WHR 101 transmits, to the management apparatus 102, a search response which is a command responsive to the search request (S5).

Next, the management apparatus 102 verifies (or detects) presence of the WHR 101 by receiving the search response from the WHR 101, and transmits, to the WHR 101, a command for request to obtain health information (S6).

Next, when the WHR 101 receives the command for request to obtain health information from the management apparatus 102, the WHR 101 transmits the held body temperature data to the management apparatus 102 (S7). At this time, a clock count at a time when the body temperature data containing the clock count at measurement is transmitted (a clock count at transmission) is transmitted with the body temperature data.

Next, the management apparatus 102 receives, from the WHR 101, the body temperature data containing the clock count at measurement and the clock count at transmission, as health information. At this time, the management apparatus 102 converts the clock count at measurement into real time information, based on the clock count at transmission received from the WHR 101 and time (current time) indicated by the time counter 305 at a time when the body temperature data and the clock count at transmission are received from the WHR 101.

In addition, the management apparatus 102 transmits, to the WHR 101, reception confirmation indicating the reception of the body temperature data from the WHR 101 (S8).

After receiving the reception confirmation from the management apparatus 102, the WHR 101 discards the held body temperature data.

It is to be noted that in the case where the reception confirmation cannot be received from the management apparatus 102 even when a certain period of time passes after having transmitted the body temperature data to the management apparatus 102, the WHR 101 retransmits the body temperature data.

As stated above, the management apparatus 102 collects, from the WHR 101, the body temperature data measured using the clinical thermometer 112, as the health information.

Moreover, the WHR 101 causes the clinical thermometer 112 to measure the body temperature by transmitting the command for request to obtain body temperature data to the clinical thermometer 112. This way, the WHR 101 obtains, as the health information, the body temperature measured by the clinical thermometer 112. As a result, it is possible to reduce a time lag between timing at which a measuring device such as the clinical thermometer 112 measures the health information and timing at which a health information collecting apparatus such as the WHR 101 obtains the health information. This enables the management apparatus 102 to accurately calculate time when the health information is measured.

In the next place, operations in which the WHR 101 obtains health information from the variety of sensors and then transmits the obtained health information to the management apparatus 102 are described. Here, a case where body temperature data measured using the clinical thermometer 112 is transmitted as health information to the management apparatus 102 is described as an example.

### <Conversion into Time>

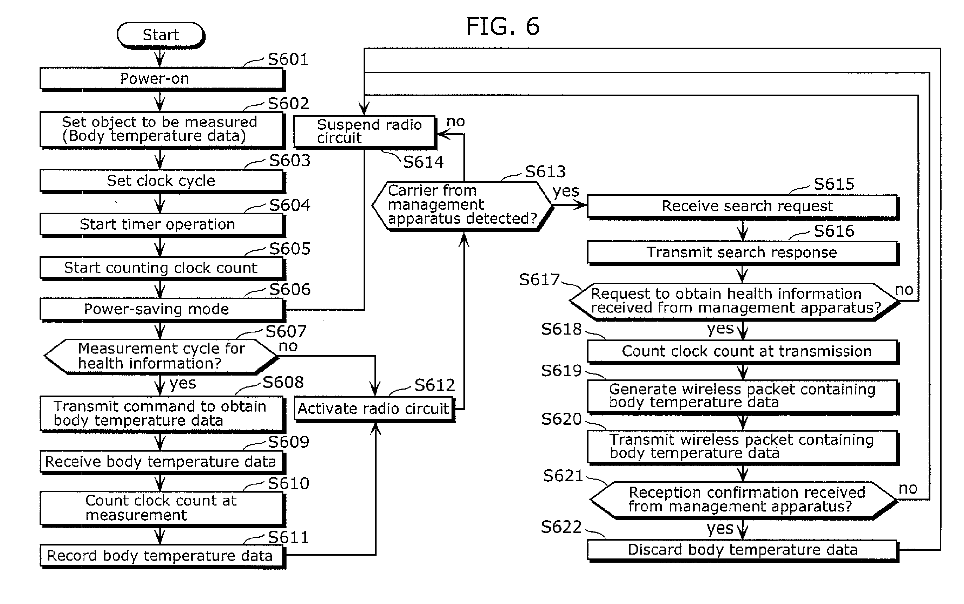

FIG. 6 is a flow chart describing operations of the WHR 101 according to the first embodiment of the present invention.

First, the user 11 turns ON the power to the WHR 101 (S601).

Next, the user 11 sets an object to be measured to the WHR 101 (S602). Here, the object to be measured is a body temperature of the user 11, and data to be obtained is body temperature data.

Next, the user 11 sets a clock cycle for the WHR 101 (S603). It is to be noted that, when transmitting the body temperature data to the management apparatus 102, the clock cycle set here is contained in a header of the body temperature data and transmitted to the management apparatus 102.

Next, the WHR 101 starts a timer operation (S604), and counting a clock (S605). That is to say, the oscillator circuit 205, the internal timer, increments a clock count as the clock is counted, for instance, for every minute, after the timer operation is started.

Next, the WHR 101 enters into a power-saving mode (S606).

Next, the WHR 101 confirms whether a measurement cycle for health information has come for timing at which health information is measured (S607). In the case where it is confirmed that the measurement cycle for health information has come (Yes in S607), the WHR 101 transmits, to the clinical thermometer 112, a command for request to obtain body temperature data (S608). Here, Step S608 is equivalent to S1 in FIG. 5.

Next, the WHR 101 receives, from the clinical thermometer 112, the body temperature data as the health information (S609). At this time, the WHR 101 determines the clock count at measurement (S610) and holds the body temperature data and the clock count at measurement by storing, in the memory 203, the determined clock count at measurement together with the body temperature data received from the clinical thermometer 112 (S611). Here, Step S609 is equivalent to S2 in FIG. 5, and Step S611 is equivalent to S3 in FIG. 5.

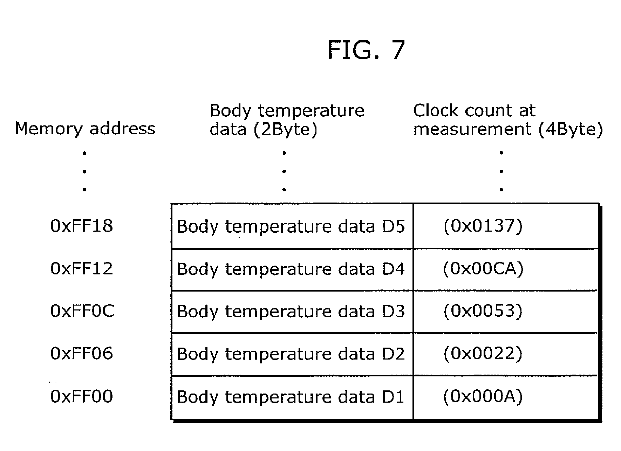

FIG. 7 illustrates a status of data stored in the memory 203 of the WHR 101 according to the first embodiment of the present invention. When the WHR 101 obtains the body temperature data from the clinical thermometer 112 as mentioned above, the WHR 101 stores, in the memory 203, the clock count at the time of the obtainment as the clock count at measurement and the obtained body temperature data, in association with each other.

As shown in FIG. 7, for example, body temperature data D1 is stored at a memory address 0 × FF00 in the memory 203, in association with 0 × 000A, a clock count at measurement at a time when the body temperature data D1 is obtained. Likewise, body temperature data D2 that is obtained after the body temperature data D1 is stored at a memory address 0 × FF06 in the memory 203, in association with 0 × 0022, a clock count at measurement at a time when the body temperature data D2 is obtained.

Next, the WHR 101 activates a radio circuit (S612), performs a carrier sense operation, and confirms whether a carrier from the management apparatus 102 is detected, that is, a command is transmitted.

In the case where the carrier (radio wave) from the management apparatus 102 can be detected (S613), the WHR 101 receives, from the management apparatus 102, a search request as an activation command (S615).

Next, the WHR 101 transmits, to the management apparatus 102, a search response that is a command for response to the search request (S616). Here, Step S615 is equivalent to S4 in FIG. 5, and Step S616 is equivalent to S5 in FIG. 5.

Next, when the WHR 101 receives, from the management apparatus 102, the command for request to obtain health information (Yes in S617), the WHR 101 determines, as the clock count at transmission, a clock count at a time when the body temperature data containing the clock count at measurement is transmitted (S618).

The WHR 101 generates a wireless communication packet for transmitting, to the management apparatus 102, the determined clock count at transmission together with the body temperature data containing the clock count at measurement (hereinafter, described as wireless packet containing body temperature data) (S619). Here, Yes in Step S617 is equivalent to S6 in FIG. 5.

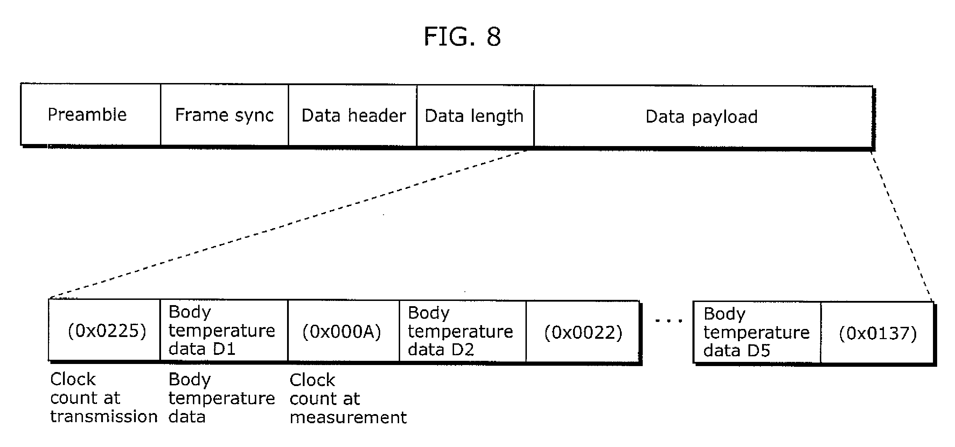

FIG. 8 illustrates a data frame at a time when the WHR 101 transmits health information to the management apparatus 102 according to the first embodiment of the present invention. The data frame at the time when the health information is transmitted is the wireless packet containing body temperature data. The wireless packet containing body temperature data includes a preamble, a frame sync, a data head, data length, and a data payload.

As shown in FIG. 8, in the case where the WHR 101 transmits, to the management apparatus 102, body temperature data as health information, a clock count (0 × 02225) at a time when a group of body temperature data is transmitted is transmitted as the clock count at transmission together with the group of body temperature data. Here, the group of body temperature data contains plural body temperature data and clock counts at measurement, and each of the clock counts at measurement is associated with corresponding one of the plural body temperature data when each body temperature data is obtained. As mentioned above, the clock cycle set at S603 is described on the data header.

The data payload includes the clock count at transmission at the beginning and, subsequent to the beginning, the group of body temperature data containing the plural body temperature data and the clock counts at measurement, each of the clock counts at measurement being associated with the corresponding one of the plural body temperature data when each body temperature data is obtained.

Next, the WHR 101 transmits, to the management apparatus 102, the wireless packet containing body temperature data generated for transmitting, to the management apparatus 102, the determined clock count at transmission together with the body temperature data containing the clock count at measurement (S620). Here, Step S620 is equivalent to S7 in FIG. 5.

Next, when the WHR 101 receives, from the management apparatus 102, reception confirmation indicating the reception of the body temperature data by the management apparatus 102 (Yes in S621), the WHR 101 discards the body temperature data containing the clock count at measurement that is held therein (S622). Here, Yes in Step S621 is equivalent to S8 in FIG. 5.

It is to be noted that in Step S613, in the case where the WHR 101 cannot detect the carrier (radio wave) from the management apparatus 102 (No in S613), the WHR 101 stops the carrier sense operation and suspends the radio circuit (S614). Subsequently, the WHR 101 enters into a suspended state, that is, enters into the power-saving mode (S606).

Furthermore, in Step S607, in the case where the WHR 101 confirms that the measurement cycle for health information has not come (No in S607), the WHR 101 activates the radio circuit (S612), performs the carrier sense operation, and confirms whether the carrier from the management apparatus 102, that is, the command has been transmitted.

As stated above, the WHR 101, a health information collecting apparatus, obtains the health information from the variety of sensors, and transmits the obtained health information to the management apparatus 102.

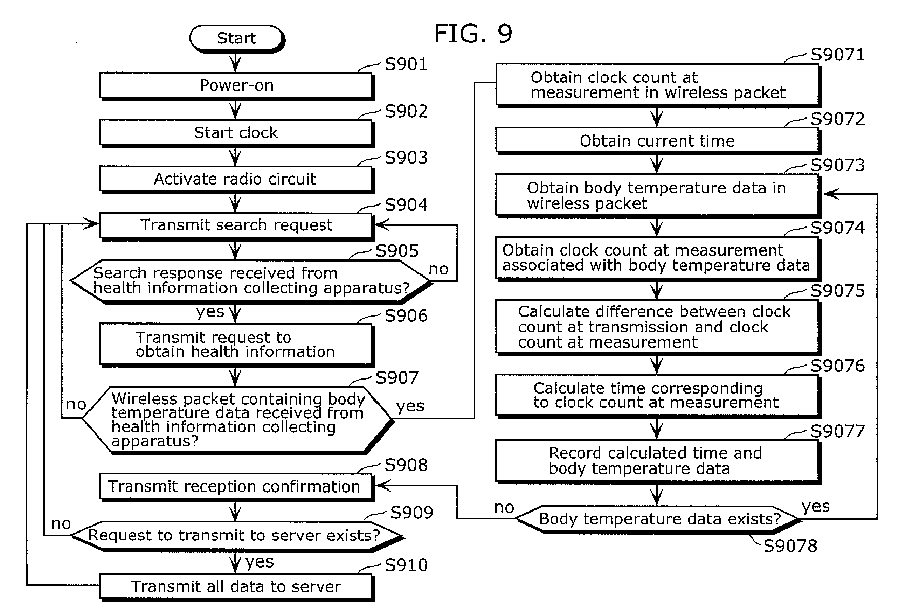

FIG. 9 is a flow chart describing operations of the management apparatus 102 according to the first embodiment of the present invention.

First, when the user 11 turns ON the power to the management apparatus 102 (S901), the management apparatus 102 starts a real time clock (S902).

Next, the management apparatus 102 activates the radio circuit (5903), and transmits a search request as an activation command to the WHR 101 (S904). Here, the search request from the management apparatus 102 is continuously transmitted for a period of time longer than a period of the suspended state of the WHR 101, a health information collecting apparatus. This allows the search request to be detected surely by the carrier sense operation performed by the WHR 101 in the case where the WHR 101 is located within a wirelessly reachable range of the management apparatus 102. Moreover, Step S904 is equivalent to S4 in FIG. 5.

Next, when the management apparatus 102 receives a search response from the WHR 101 (Yes in S905), the management apparatus 102 verifies presence of the WHR 101 and transmits a command for request to obtain health information to the WHR 101 (S906). Here, Yes in Step S905 is equivalent to S5 in FIG. 5, and Step S906 is equivalent to S6 in FIG. 5.

Next, the management apparatus 102 receives, from the WHR 101, a wireless packet containing body temperature data containing both the clock count at transmission and body temperature data containing the clock count at measurement, as the health information (Yes in S907). After Steps S9071 to S9078, the management apparatus 102 transmits reception confirmation to the WHR 101 (S909).

Here, Steps S9071 to S9078 are described.

When the management apparatus 102 receives, from the WHR 101, the wireless packet containing body temperature data (Yes in S907), the management apparatus 102 obtains the clock count at transmission contained in the wireless packet containing body temperature data (S9071). In addition, the management apparatus 102 obtains time (current time) indicated by the time counter 305 at a time when the wireless packet containing body temperature data is received (S9072).

Next, the management apparatus 102 obtains the body temperature data contained in the wireless packet containing body temperature data (S9073) and the clock count at measurement associated with the body temperature data contained in the wireless packet containing body temperature data (S9074).

Next, the management apparatus 102 calculates a difference between the clock count at transmission and the clock count at measurement obtained (S9075) and then time corresponding to the clock count at measurement based on the current time (S9076).

Next, the management apparatus 102 records the calculated time and the body temperature data, in association with each other (S9077).

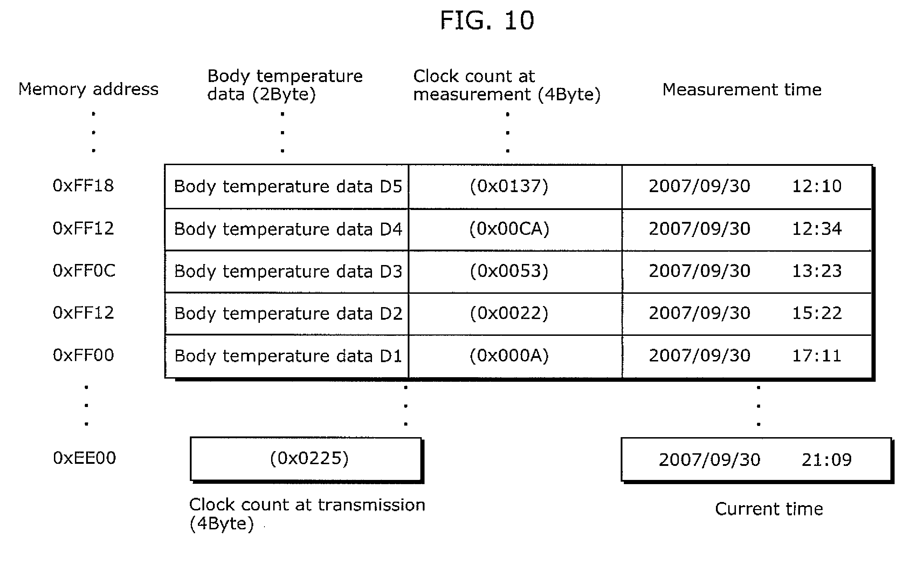

FIG. 10 illustrates conversion of a clock count at body temperature measurement to time according to the first embodiment of the present invention.

The management apparatus 102 receives the wireless packet containing body temperature data, and associates the clock count at transmission with the current time, that is, real time information at a time of receiving the wireless packet containing body temperature data, when the management apparatus 102 obtains a clock count at transmission and a group of body temperature data containing plural body temperature data each containing a clock count at measurement.

For instance, as shown in FIG. 10, clock count at transmission (0 × 0225) is associated with the current time "2007/09/30 21:09".

In the management apparatus 102, a clock cycle period (one minute) of the WHR 101 is known. Here, the clock cycle period is described on a data header of a wireless packet containing body temperature data, and obtained from the data header of the wireless packet containing body temperature data received by the management apparatus 102.

The management apparatus 102 calculates time corresponding to the clock count at measurement associated with each body temperature data, using a difference between the clock count at transmission and the clock count at measurement, based on the current time corresponding to the clock count at transmission.

A result of converting the clock count at measurement to the time is as shown in FIG. 10, and, for example, time when the WHR 101 obtained body temperature data D1 is calculated as "2007/09/30 12:10".

Next, after recording, as the health information, the time calculated as mentioned above and the body temperature data, in association with each other (S9077), the management apparatus 102 confirms whether extra body temperature data exists in the obtained wireless packet containing body temperature data (S9078). Here, in the case where the management apparatus 102 confirms that the extra body temperature data exists in the obtained wireless packet containing body temperature data (Yes in S9078), the management apparatus 102 repeats Steps S9073 to S9077.

Next, in the case where the management apparatus 102 confirms that no further body temperature data exists in the obtained wireless packet containing body temperature data (No in S9078), the management apparatus 102 transmits the reception confirmation indicating the reception of the body temperature data from the WHR 101 (S908). Here, Step S908 is equivalent to S8 in FIG. 5.

Next, the management apparatus 102 confirms whether a request to transmit to the information accumulating server 105 exists (S909). In the case where the management apparatus 102 confirms that the request to transmit to the information accumulating server 105 exists (Yes in S909), the management apparatus 102 transmits, to the information accumulating server 105, all of the health information held (here, the body temperature data) (S910).

After transmitting the health information (here, the body temperature data) to the information accumulating server 105, the management apparatus 102 restarts operations from Step S904.

It is to be noted that in Step S907, in the case where the management apparatus 102 does not receive the wireless packet containing body temperature data from the WHR 101 (No in S907), the management apparatus 102 restarts the operations from Step S904.

In addition, in Step S909, in the case where the request to transmit to the information accumulating server 105 exists (Yes in S909), the management apparatus 102 restarts the operations from Step S904 likewise.

As stated above, the management apparatus 102 obtains the health information from the WHR 101, a health information collecting apparatus, and transmits the obtained health information to the information accumulating server 105.

Thus, according to the first embodiment of the present invention, in the WHR 101 having no real time information, the health information measured by the sensor is associated with the clock count at measurement, and, when further transmitting to the management apparatus 102, the associated health information is transmitted, to the management apparatus 102, together with the clock count at transmission at the time of the transmission. In the management apparatus 102, the health information is received from the WHR 101, the clock count at transmission is converted to real time information based on the current time when the health information is received from the WHR 101, and time when the health information is measured is further calculated using the difference between the clock count at transmission and the clock count at measurement.

Accordingly, it is possible to realize the health information collecting apparatus, the management apparatus, and the health information collecting system which can determine the obtainment time of the health information collected using the battery-driven wearable terminal having low operation specifications and no real time clock.

### (Second Embodiment)

In the first embodiment, the clinical thermometer 112 is exemplified as the sensor, and the example where the user 11 carries the clinical thermometer 112 with the WHR 101.

In the second embodiment, a weighing scales 107 is exemplified as a sensor that the user 11 does not carry, and a case where the WHR 101 carried by the user 11 obtains health information from the sensor.

### <Terminal ID Setting>

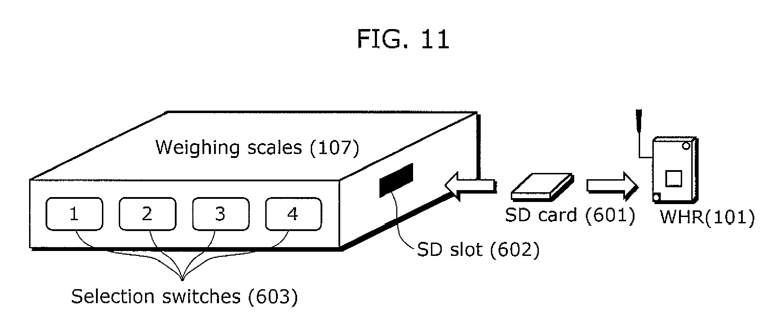

FIG. 11 illustrates setting of a terminal ID to a weighing scales, a sensor, according to a second embodiment of the present invention. In FIG. 11, the same elements as in FIG. 2 are indicated by the same reference numerals, and their description is omitted.

The weighing scales 107, a sensor, has selection switches 603 and a SD slot 602 to which a SD card 601 is inserted, in addition to a weight measuring function.

Each of numerals 1 to 4 is assigned to a corresponding selection switch 603, and a selection switch 603 can be selected with a user's simple operation such as the use of a foot.

The SD card 601 contains a terminal ID of the WHR 101, and sets the terminal ID inside the weighing scales 107 by inserting the SD card 601 to the SD slot 602.

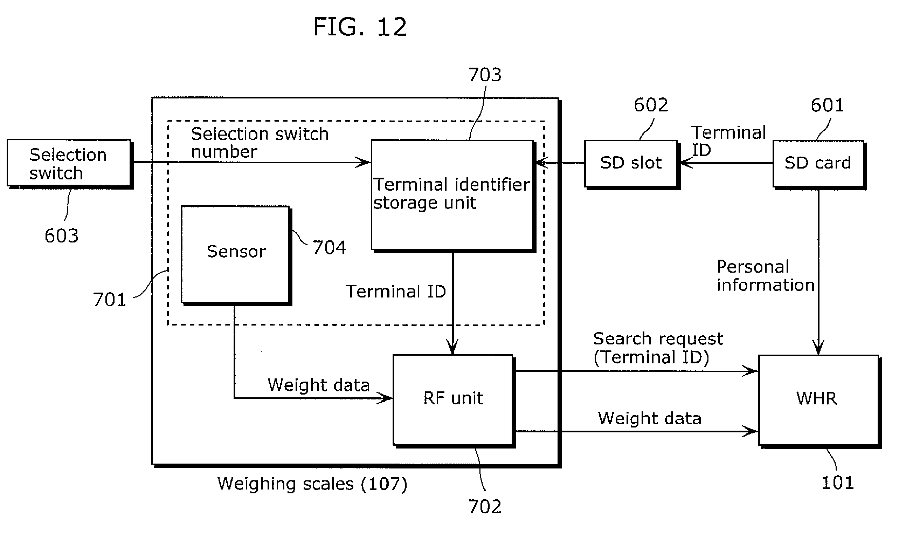

FIG. 12 is a block diagram illustrating a configuration in the case where a SD card is inserted and attached to the weighing scales according to the second embodiment of the present invention. In FIG. 12, the same elements as in FIG. 2 are indicated by the same reference numerals, and their description is omitted. FIG. 12 illustrates the configuration of the weighing scales at a time when the SD card 601 is attached and the terminal ID is set.

The weighing scales 107 includes a weighing scales sensor unit 701 and an RF unit 702.

The weighing scales sensor unit 701 includes a terminal identifier storage unit 703 and a sensor 704 which measures weight of the user 11.

The terminal identifier storage unit 703 stores any one of the numerals 1 to 4 selected by operating a selection switch 603.

Here, since the SD card 601 is inserted to the SD slot 602 and attached to the weighing scales 107, the terminal identifier storage unit 703 extracts the terminal ID contained in the SD card 601, and stores the numeral selected by the selection switch 603 and the extracted terminal ID, in association with each other.

It is to be noted that the SD card 601 may encrypt personal information and contain the encrypted personal information, and may be used as a medium for providing the personal information to the WHR 101 by inserting the SD card 601 to the WHR 101.

### <Communication Procedures>

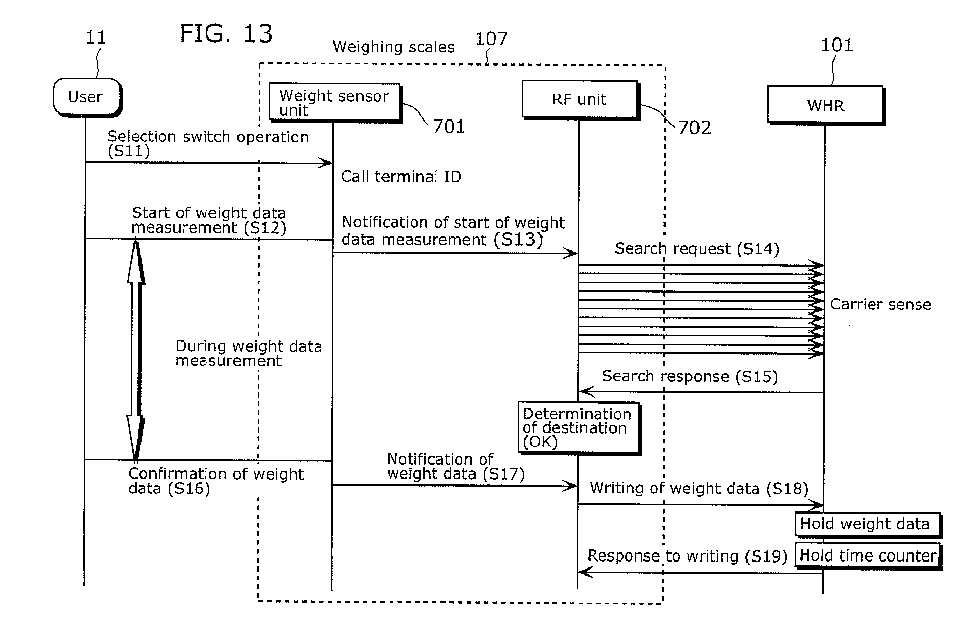

FIG. 13 illustrates WHR 101-weighing scales 107 communication procedures at weight measurement according to the second embodiment of the present invention. In FIG. 13, the same elements as in FIGS. 2 and 12 are indicated by the same reference numerals, and their description is omitted.

Personal information (gender, height, age, etc.) and numerals of the selection switches 603 relating to the personal information are set to the weighing scales 107 beforehand. In addition, the SD card 601 is attached to and a terminal ID is set to the weighing scales 107, and the terminal ID and a numeral of a selection switch 603 included in the weighing scales 107 are associated.

First, the user 11 operates a selection switch 603 to select a numeral with which information of the user 11 is registered (S11).

The selection switch 603 calls the terminal ID of the WHR 101 according to the numeral selected by the user 11.

Next, when the user 11 steps on the weighing scales 107, the sensor 704 in the weighing scales sensor unit 701 is activated to start measuring the weight of the user 11 (S12).

When the weight measurement is started, the sensor 704 in the weighing scales sensor unit 701 transmits, to the RF unit 702, a weight measurement start notification indicating that the weight measurement is started (S13).

Next, when receiving the weight measurement start notification, the RF unit 702 transmits, to the WHR 101, a search request as an activation command (S14). It is to be noted that the search request contains the terminal ID called in Step S11.

The WHR 101 detects the search request from the weighing scales 107 by performing a carrier sense operation. When detecting the search request from the weighing scales 107, the WHR 101 determines whether the search request contains a self-terminal ID of the WHR 101. It is to be noted that in the case where the search request does not contain the self-terminal ID of the WHR 101, the WHR 101 performs the intermittent operation.

Next, in the case where the search request contains the self-terminal ID of the WHR 101, the WHR 101 transmits, to the weighing scales 107, a search response which is a response to the search request from the weighing scales 107 (S15). The weighing scales 107 verifies (or detects) presence of the WHR 101 by receiving the search response from the WHR 101. Here, the weighing scales 107 determines a destination by verifying the terminal ID of the WHR 101.

Next, the sensor 704 in the weighing scales sensor unit 701 completes the weight measurement of the user 11 and confirms weight data (S16).

Next, when the sensor 704 in the weighing scales sensor unit 701 confirms the weight data, the sensor 704 transmits the weight data to the RF unit 702 (S17).

Next, the weighing scales 107 transmits the weight data and a command to write the weight data to the WHR 101 via the RF unit 702 (S18).

The WHR 101 receives, from the weighing scales 107, the weight data and the command to write the weight data, and holds the weight data received from the weighing scales 107 and a clock count at measurement which is a clock count at a time when the weight data is received.

Next, after holding the weight data received from the weighing scales 107 and the clock count at measurement, the WHR 101 transmits, to the weighing scales 107, a response to writing which is a response to the command to write the weight data (S19).

As stated above, the WHR 101 obtains the weight data as the health information from the weighing scales 107. Accordingly, even when, for example, two WHRs 101 are located near the weighing scales 107, by selecting the numeral assigned to the user 11 with the selection switch 603, it is possible to correctly write the weight data to the WHR 101 carried by the user 11 oneself.

It is to be noted that, as mentioned above, the WHR 101 obtains the weight data as the health information from the weighing scales 107, a measuring device, after an event in which the user 11 uses the weighing scales 107 for weight measurement occurs and the weighing scales 107 verifies the presence of the WHR 101 in Step S5. Although there is a time lag between timing at which the measuring device such as the weighing scales 107 measures health information and timing at which a health information collecting apparatus such as the WHR 101 obtains the health information, this is not an issue since it is not necessary to be accurate in time counted by minutes for weight measurement. Thus, the management apparatus 102 can accurately calculate time when the health information is measured.

As described above, according to the present invention, it is possible to realize the health information collecting apparatus, the management apparatus, and the health information collecting system which can determine the obtainment time of the health information collected using the battery-driven wearable terminal having low operation specifications and no real time clock.

### (Further Notes)

### <Measurement of Wireless Communication Frequency>

It is to be noted that a wireless communication frequency measurement unit may be provided for the WHR 101, so as to measure a wireless communication frequency indicating how many times wireless communication between the WHR 101 and the management apparatus 102 is performed in a predetermined period of time (for example, a day) and to set a sensor communication frequency at which pedometer data is obtained from a sensor such as the pedometer 111 according to the wireless communication frequency.

In such case, when the wireless communication frequency between the WHR 101 and the management apparatus 102 is more than a predetermined number of times, the sensor communication frequency at which health information is obtained from the pedometer 111 is increased. Furthermore, when the wireless communication frequency between the WHR 101 and the management apparatus 102 is fewer than a predetermined number of times, the sensor communication frequency at which health information is obtained from the pedometer 111 is decreased. For instance, when it is observed that the wireless communication frequency is more than five times a day, the sensor communication frequency is increased from once every 10 minutes to once every seven minutes and further to once every five minutes; or when it is not observed that the wireless communication frequency is once a day, the sensor communication frequency is decreased from once every 10 minutes to once every 15 minutes and further to once every 20 minutes.

In addition, when the sensor communication frequency is higher or lower than a specific value, an upper or lower limit may be set to the sensor communication frequency so that the wireless communication frequency is not changed.

Moreover, the wireless communication frequency measurement unit may be provided for the management apparatus 102, so as to set a notification frequency to the WHR 101 according to a wireless communication frequency, the notification frequency being from the WHR 101 to the management apparatus 102.

### <Monitoring Storage Capacity>

Furthermore, a storage capacity monitoring unit may be provided for the WHR 101 so as to monitor a remaining storage capacity of the memory 203 of the WHR 101. In such case, when the remaining storage capacity of the memory 203 of the WHR 101 becomes lower than a predetermined proportion, a sensor communication frequency at which health information is obtained from a sensor such as the pedometer 111 is decreased. For example, when the remaining storage capacity becomes lower than 30 percent, the sensor communication frequency is changed from once every 10 minutes to once every 15 minutes.

Here, when the sensor communication frequency is lower than a specific value of a storage capacity, an upper or lower limit may be set to the sensor communication frequency so that a wireless communication frequency is not changed.

Since this allows control of a health information obtainment frequency in a sensor, it is possible to effectively use the memory 203 of the WHR 101.

### <Information Compression>

Moreover, an information compression unit may be provided for the WHR 101 so as to perform information compression on health information held and a clock count at measurement. In such case, for example, a remaining storage capacity of the memory 203 may be monitored, and the information compression may be performed when the remaining storage capacity becomes lower than a predetermined proportion.

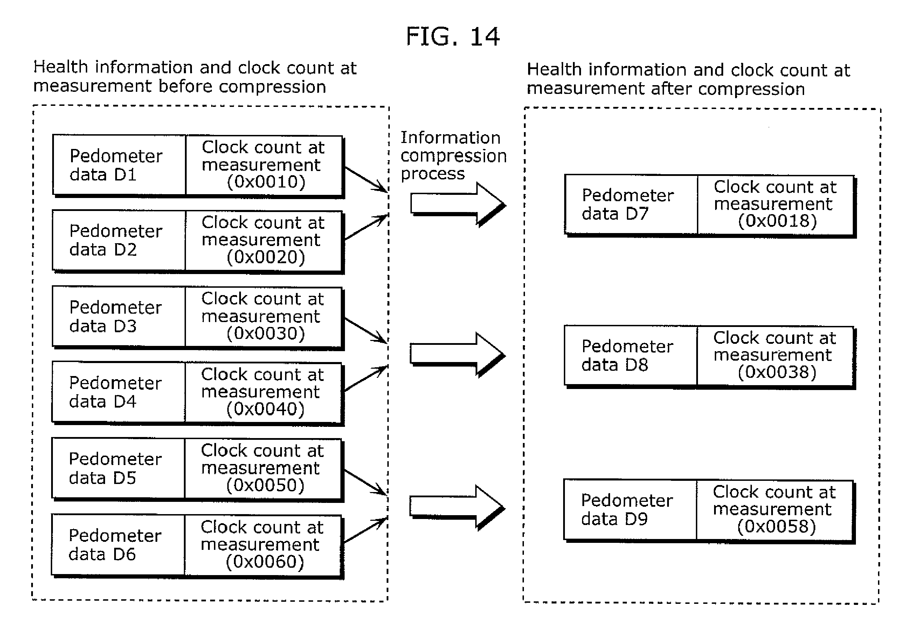

FIG. 14 illustrates one example of information compression at pedometer measurement according to the present invention. As shown in the left-hand side of FIG. 14, the WHR 101 holds pedometer data D1 to D6 obtained from, for example, a sensor of the pedometer 111 and a clock count at measurement associated with each pedometer data. Here, health information and the clock count at measurement, that is, each pedometer data and the clock count at measurement associated with each pedometer data are not compressed. When, however, a remaining storage capacity of the memory 203 becomes less than a predetermined proportion, the information compression unit performs information compression. As shown in the right-hand side of FIG. 14, the information compression is performed on plural health information and plural clock counts at measurement by converting plural pedometer data and the clock counts at measurement each associated with corresponding one of the plural pedometer data into several pedometer data each associated with one clock count, the several pedometer data being generated by grouping the plural pedometer data.

An example of an information compression method includes averaging. For instance, in the case where pedometer data D1 and pedometer data D2 are converted to pedometer data D7, an average value between pedometer data D1 and pedometer data D2 is pedometer data D7. In the example shown in FIG. 14, clock count at measurement "0 × 0010" relating to pedometer data D1 and clock count at measurement "0 × 0020" relating to pedometer data D2 are averaged to generate clock count at measurement "0 × 0018" relating to pedometer data D7. It is to be noted that the information compression method may be not only the above-mentioned averaging method but also a calculation method in which a measurement time interval of each data is weighted.

Accordingly, it becomes possible to effectively use the memory 203 of the WHR 101.

Furthermore, every time health information is obtained, a health information collecting apparatus such as the WHR 101 may transmit, with a clock count at transmission, a clock count at measurement and the obtained health information, or transmit, with a count clock at transmission, sets of health information and a clock count associated with the health information. Transmitting the sets of the health information and the clock count all together to a management apparatus not only simplifies processing as a whole but also allows calculation of a measurement time of each of plural health information based on the clock count at transmission.

In addition, the health information collecting apparatus such as the WHR 101 may transmit, as a clock count at measurement, a difference clock count between the clock count at transmission and the clock count at measurement (hereinafter, described as a difference clock count at measurement), the clock count at transmission being the basis for calculating the difference clock count at measurement. In such case, the health information collecting apparatus may transmit, to the management apparatus, the clock count at transmission together with health information and the difference clock count at measurement associated with the health information.

Accordingly, the structure of the health information collecting apparatus can be simplified, and a physical and mental strain on the human body when the health information collecting apparatus is carried can be reduced. Moreover, since data amount of data transmitted by the health information collecting apparatus and data amount of data received by the management apparatus can be reduced by transmitting the difference clock count at measurement as the clock count at measurement, a processing load of the health information collecting apparatus and the management apparatus can be reduced.

Although the health information collecting apparatus, the management apparatus, and the health information collecting system, and the method for collecting health information according to the present invention have been described based on the embodiments above, the present invention is not limited to the embodiments. Those skilled in the art will readily appreciate that many modifications are possible in the exemplary embodiments without materially departing from the novel teachings and advantages of this invention. Accordingly, all such modifications are intended to be included within the scope of this invention.

### INDUSTRIAL APPLICABILITY

The health information collecting system according to the present invention is useful as a health information collecting system which collects health information using a wearable wireless device having too low operation specifications to manage real time information as well as manages real time information on obtainment of the health information.

## Claims

1. A health information collecting apparatus which obtains health information from a measuring device and transmits the obtained health information to a management apparatus, the measuring device measuring the health information of a user of the measuring device, the management apparatus managing the transmitted health information, said health information collecting apparatus comprising:
a clock unit configured to oscillate a clock;
a counting unit configured to count the clock oscillated by said clock unit;
an obtainment unit configured to receive the health information from the measuring device;
a holding unit configured to hold the health information and a first clock count associated with the health information, the first clock count being counted by said counting unit when the health information is received; and
a transmission unit configured to transmit, to the management apparatus, the health information and the first clock count associated with the health information,
wherein said transmission unit is configured to transmit, to the management apparatus, a second clock count with the health information and the first clock count, the second clock count being counted by said counting unit when the health information and the first clock count are transmitted to the management apparatus.

2. The health information collecting apparatus according to Claim 1,
wherein said transmission unit is configured to transmit, to the management device, the health information and the first clock count, with clock cycle information indicating a time interval of a clock cycle at which said clock unit oscillates the clock.

3. The health information collecting apparatus according to Claim 2, further comprising
a carrier sense unit configured to detect a radio wave,
wherein said transmission unit is configured to transmit the health information and the first clock count with the second clock count, when the management apparatus requests said health information collecting apparatus for the health information and when said carrier sense unit detects a radio wave from the management apparatus.

4. The health information collecting apparatus according to Claim 3,
wherein said obtainment unit is configured to request the measuring device to transmit, to said health information collecting apparatus, the health information measured by the measuring device, and to receive, from the measuring device, the health information.

5. The health information collecting apparatus according to Claim 4,
wherein said obtainment unit is configured to request the measuring device for the health information, when the measuring device detects said health information collecting apparatus and when said carrier sense unit detects the radio wave from the measuring device.

6. The health information collecting apparatus according to Claim 5,
wherein said holding unit is configured to hold a plurality of sets of the health information and the first clock count, and to hold an interspace of time as the first clock count associated with health information, the interspace of time being a difference between a previous clock count counted by said counting unit at a time when previous health information is received by said health information collecting apparatus and a current clock count counted by said counting unit at a time when current health information is received by said health information collecting apparatus, and
said transmission unit is configured to transmit, to the management apparatus, with the plurality of sets of the health information and the interspace of time as the first clock count, a clock count as the second clock count, the clock count being counted by said counting unit at a time when the plurality of the sets of the health information and the first clock count are transmitted to the management apparatus.

7. The health information collecting apparatus according to Claim 6, further comprising:
a storage capacity detection unit configured to detect a remaining storage capacity of said holding unit; and
an information compression unit configured to compress specific sets of the health information and the first clock count among the sets of the health information and the first clock count,
wherein said information compression unit is configured to compress the specific sets of the health information and the first clock count, when said storage capacity detection unit detects that the remaining storage capacity is equal to or less than a predetermined value.

8. The health information collecting apparatus according to Claim 4,
wherein said health information collecting apparatus includes the measuring device.

9. The health information collecting apparatus according to Claim 8,
wherein the health information comprises a body temperature, a blood pressure, a cardiogram, a blood-sugar level, and the number of walking steps taken.

10. The health information collecting apparatus according to Claim 1,
wherein said transmission unit is configured to transmit, to the management apparatus, the second clock count with the health information and a third clock count as the first clock count, the third clock count being a difference between the first clock count and the second clock count, the second clock count being the basis for calculating the difference.

11. A management apparatus for managing health information which receives the health information from a health information collecting apparatus for collecting the health information measured by a measuring device, the measuring device measuring the health information, said management apparatus comprising:
a communication unit configured to receive the health information, a first clock count and a second clock count from the health information collecting apparatus, the first clock count being a clock count value at a time when the health information collecting apparatus collects the health information from the measuring device , the second clock count being another clock count value at a time when the health information collecting apparatus transmits, to the management apparatus, the health information and the first clock count associated with the health information;
a holding unit configured to hold the health information, the first clock count associated with the health information, and the second clock count;
a time determination unit configured to determine a reception time of receiving the second clock count; and
a conversion unit configured to convert the first clock count to actual time using a difference between the first clock count and the second clock count, based on the reception time determined by said time determination unit, and to hold the actual time with the health information.

12. The management apparatus according to Claim 11,
wherein said communication unit is configured to receive, from the health information collecting apparatus, the health information, the second clock count, and a third clock count as the first clock count, the third clock count being a difference between the first clock count and the second clock count, the second clock count being the basis for calculating the difference, and
said conversion unit is configured to convert the third clock count to actual time using the difference, based on the reception time of receiving the second clock count determined by said time determination unit, and to hold the actual time with the associated health information.

13. A method for collecting health information with which the health information is obtained from a measuring device, the measuring device measuring the health information, the obtained health information being transmitted to a management apparatus for managing the health information, said method comprising:
oscillating a clock;
counting the clock oscillated in said oscillating;
receiving the health information from the measuring device;
holding the health information and a first clock count with the health information, the first clock count being counted when the health information is received; and
transmitting, to the management apparatus, the health information and the first clock count,
wherein a second clock count is transmitted with the health information and the first clock count to the management apparatus, the second clock count being counted when the health information and the first clock count are transmitted to the management apparatus.

14. A computer program product for collecting health information with which the health information is received from a measuring device, the measuring device measuring the health information, the obtained health information being transmitted to a management apparatus for managing the health information, said computer program product, when loaded into a computer, allowing the computer to execute:
oscillating a clock;
counting the clock oscillated in said oscillating;
receiving the health information by from the measuring device;
holding the health information and a first clock count with the health information, the first clock count being counted when the health information is received; and
transmitting, to the management apparatus, the health information and the first clock count associated with the health information,
wherein a second clock count is transmitted with the health information and the first clock count to the management apparatus, the second clock count being counted when the health information and the first clock count are transmitted to the management apparatus.

15. A health information collecting system comprising a health information collecting apparatus for collecting the health information from a measuring device, the measuring device measuring the health information, and a management apparatus for receiving the health information from the health information collecting apparatus and for managing the health information,
wherein said health information collecting apparatus includes:
a clock unit configured to oscillate a clock;
a counting unit configured to count the clock oscillated by said clock unit;
an obtainment unit configured to receive the health information from the measuring device;
a holding unit configured to hold the health information and a first clock count associated with the health information, the first clock count being counted by said counting unit when the health information is received; and
a transmission unit configured to transmit, to said management apparatus, the health information and the first clock count associated with the health information,
wherein said transmission unit is configured to transmit, to said management apparatus, a second clock count with the health information and the first clock count, the second clock count being counted by said counting unit when the health information and the first clock count are transmitted to said management apparatus, and said management apparatus includes:
a communication unit configured to receive, from said health information collecting apparatus, the health information, the first clock count associated with the health information, and the second clock count;
a holding unit configured to hold the health information, the first clock count, and the second clock count;
a time determination unit configured to determine a reception time of receiving the second clock count; and
a conversion unit configured to convert the first clock count to actual time using a difference between the first clock count and the second clock count, based on the reception time determined by said time determination unit, and to hold the actual time and the health information associated with the actual time.

16. A health information collecting apparatus comprising:
a clock unit configured to oscillate a clock;
a counting unit configured to count the clock;
an interface unit configured to communicate with a measuring device for measuring health information; and
a control unit configured to:
count a first clock count at a time when the health information is received from the measuring device;
count a second clock count at a time when the health information is transmitted to a management apparatus, the management apparatus managing the health information;
associate the health information with the first clock count and the second clock count; and
transmit, to the management apparatus, the health information associated with the first clock count and the second clock count, the first clock count, and the second clock count.

17. The health information collecting apparatus according to Claim 16, further comprising
a storage unit configured to store a time interval in the case of measuring the health information, the health information measured by the measuring device at every time interval, and the first clock count at each time when the health information is received,
wherein said control unit is configured to transmit, to the management apparatus, the health information measured at every time interval, the first clock count at each time when each health information is received, and the second clock count at each time when each health information is transmitted.

18. A management apparatus comprising:
a communication unit configured to receive, from a health information collecting apparatus, health information, a first clock count at a time when the health information is measured, and a second clock count at a time when the health information is transmitted, the health information collecting apparatus collecting the health information from a measuring device, the measuring device measuring the health information of a user of the measuring device;
a storage unit configured to store the health information;
a time determination unit configured to determine a reception time of receiving the health information; and
a control unit configured to calculate a measurement time of measuring the health information, based on a difference between the first clock count , the second clock count and the reception time, and to store, in said storage unit, the measurement time, in association with the health information.

19. A information collecting system comprising a health information collecting apparatus, a measuring device and a management apparatus, the health information collecting apparatus receiving health information from the measuring device, the measuring device measuring the health information, the management apparatus receiving the health information from said health information collecting apparatus and managing the received health information,
wherein said health information collecting apparatus includes:
a clock unit configured to oscillate a clock;
a counting unit configured to count the clock;
an interface unit configured to communicate with the measuring device; and
a first control unit configured to count a first clock count at a time when the health information is received from the measuring device and a second clock count at a time when the health information is transmitted to the management apparatus, to associate the health information with the first clock count and the second clock count, and to transmit, to the management apparatus, the health information associated with the first clock count and the second clock count, the first clock count, and the second clock count, and
said management apparatus includes:
a communication unit configured to receive, from the health information collecting apparatus, the health information, the first clock count at the time when the health information is measured, and the second clock count at the time when the health information is transmitted;
a storage unit configured to store the health information;
a time determination unit configured to determine a reception time of receiving the health information; and
a second control unit configured to calculate a measurement time of measuring the health information based on a difference between the first clock count, the second clock count and the reception time, and to store, in said storage unit, the measurement time, in association with the health information.
